# EUROPEAN PATENT APPLICATION

(11) **EP 4 502 166 A1**
(43) Date of publication of application: **05.02.2025**
(21) Application number: 23775069.0
(22) Date of filing: 24.03.2023
(51) Int. Cl.: C12N 15/864, C12N 5/10, C12N 7/01, C12N 15/35

(54) **EFFICIENT SYSTEM FOR PRODUCING ADENO-ASSOCIATED VIRUS (AAV) VECTOR**

(30) Priority: 25.03.2022 JP 2022050212
(71) Applicant: Jichi Medical University, Tokyo 102-0093 (JP); Takara Bio Inc., Kusatsu-shi Shiga 525-0058 (JP)
(72) Inventor: OHBA, Kenji, Shimotsuke-shi, Tochigi 329-0498 (JP); ENOKI, Tatsuji, Kusatsu-shi, Shiga 525-0058 (JP)
(74) Representative: Zwicker, Jörk
(86) International application number: PCT/JP2023/011709
(87) International publication number: WO 2023/182476

(57) **Abstract**

The present invention provides a nucleic acid construct or the like containing an inducible promoter and a sequence for encoding an adeno-associated virus (AAV) Cap protein that is operably linked to the promoter.

## Description

### TECHNICAL FIELD

The present invention relates to a method for producing a vector of a virus having no envelope (hereinafter, referred to as a non-enveloped viral vector), preferably an adeno-associated viral (hereinafter, referred to as AAV) vector.

### BACKGROUND ART

Currently, in the fields of gene recombination and medicine, for gene introduction into mammalian cells including human cells, there are physical methods comprising electroporation or use of metal particles, chemical methods comprising use of nucleic acids, polycations, or liposomes, and biological methods comprising use of vectors for virus-derived gene transfer (hereinafter, referred to as viral vectors). The viral vector means a vector obtained by modifying a naturally occurring virus to enable transfer of a desired gene, or the like into a target cell via the virus. The technological development of viral vectors has progressed in recent years. Viral vectors produced using gene recombination technology are generally called recombinant viral vectors. Well-known examples of viruses from which such recombinant viral vectors are derived include enveloped viruses including retroviruses, lentiviruses, Sendai viruses, and herpes viruses, and non-enveloped viruses including adenoviruses and AAV.

Since AAV is capable of infecting cells of a wide variety of species including human, and even non-dividing cells that have completed differentiation such as blood cells, muscles and nerve cells, and is not pathogenic to human and thus has little risk of side effects, and the virus particles are physicochemically stable, usefulness of AAV as a gene transfer vector to be used in gene therapy for congenital genetic diseases as well as cancer and infectious diseases has especially attracted attention in recent years.

A typical method for producing recombinant virus vectors comprises separately introducing, of essential elements for virus particle formation, elements that have to be supplied in cis and elements that may be supplied in trans into a cell that can be used as a host, so as to prevent production of wild-type viruses and self-replication of the recombinant virus in the infected host cell. The essential elements for virus particle formation are usually introduced in the form of nucleic acid constructs into the cell to prepare a cell capable of producing a virus (hereinafter, referred to as a virus producer cell). When the cell is cultured and all the essential elements for virus particle formation are expressed in the cell, a viral vector is produced.

After virus production is achieved, the virus producer cells are collected and homogenized, and the resulting cell homogenate is subjected to filter filtration, ultracentrifugation, chromatography, ultrafiltration or the like to finally prepare the purified recombinant virus vector.

In order to reduce the price of gene therapeutic drugs comprising AAV vectors for clinical use, it is necessary to reduce manufacturing costs. Furthermore, in order to administer AAV vectors clinically, it is necessary to prepare a large amount of the AAV vectors. Thus, there is a need for development of a system that can produce a greater amount of AAV vectors than current systems. In addition, since the current systems produce a large amount of "immature AAV vectors" that do not have the AAV genome, the current systems require a very strict purification process, which is an obstacle to reducing the price of gene therapeutic drugs. Thus, there is also a need for development of a system that can efficiently produce "mature AAV vectors" having the AAV genome.

Viral vectors are useful tools in gene therapy. For peaceful and safe use of viruses including gene therapy, various viral vectors including AAV vectors and lentiviral vectors have been developed. The development of AAV vectors and lentiviral vectors has particularly progressed, and vector production systems in which viral genes are separated for safe use have been established. On the other hand, separation of the viral genes results in deviations from the original life cycle of the virus, which leads to a decrease in the viral vector production and a decrease in the proportion of mature viral vectors. Regarding the production of immature viral vectors, various studies for a variety of viruses have reported that when a gene of a viral capsid (hereinafter, referred to as "Cap") protein is expressed alone, the Cap protein forms virus-like particles (hereinafter, referred to as "VLPs"). That is, separating viral genes in a viral vector production system to increase safety ultimately leads to mass production of immature viral vectors (such as VLPs). Thus, there are needs for construction of a novel viral vector production system based on understanding of viral life cycle and construction of a next-generation viral vector production system that produces viral vectors in an increased amount and produces mature viral vectors in an increased proportion.

Among the elements necessary for AAV vector production, Rep proteins are known to exhibit cytotoxicity (Non-Patent Document 1). Thus, there have been many attempts to improve AAV vector production by regulating the expression of cytotoxic Rep proteins. For example, Zolotukhin et al. disclose a method for producing an AAV vector comprising use of an inducer element and a rep gene (Patent Document 1). On the other hand, there have been few reports of attempts to improve AAV vector production by regulating the expression of Cap proteins.

### PRIOR ART DOCUMENT

### PATENT DOCUMENT

Patent Document 1: WO2010/114948

### NON-PATENT DOCUMENT

Non-patent Document 1: J Virol. 2000 Oct; 74(20): 9441-50

### SUMMARY OF THE INVENTION

### PROBLEMS TO BE SOLVED BY THE INVENTION

As described above, there have been few attempts to improve AAV vector production by regulating the expression of Cap proteins. Thus, there is still room for the improvement.

### SOLUTIONS TO THE PROBLEMS

The present inventors intensively studied with the aim of constructing a next-generation AAV vector production system that produces AAV vectors in an increased amount and produces mature AAV vectors in an increased proportion. As a result, the present inventors found that AAV vectors can be efficiently produced by using a nucleic acid construct comprising an inducible promoter and an AAV Cap protein-coding sequence operably linked to the promoter. Furthermore, the present inventors found that AAV vectors can be efficiently produced by using a nucleic acid construct comprising two or more YY1 binding sequences, an AAV Rep protein-coding sequence, and an AAV Cap protein-coding sequence. Thus, the present invention was completed.

Specifically, the present invention relates to:
[1] a nucleic acid construct comprising an inducible promoter and an adeno-associated virus (AAV) Cap protein-coding sequence operably linked to the promoter;
[2] the nucleic acid construct according to [1], wherein the inducible promoter is a promoter to which a tetracycline response element sequence is operably linked;
[3] a cell comprising the nucleic acid construct according to [1] or [2];
[4] the cell according to [3], wherein the cell is derived from a human 293 cell;
[5] a method for producing a recombinant AAV vector, the method comprising:
   (a) culturing an adeno-associated virus (AAV) producer cell, wherein the AAV producer cell comprises a nucleic acid construct comprising an inducible promoter and an AAV Cap protein-coding sequence operably linked to the promoter;
   (b) inducing the expression of the AAV Cap protein in the cell obtained by step (a); and
   (c) culturing the cell obtained by step (b) to produce a recombinant AAV vector;
[6] the method according to [5], wherein the inducible promoter is a promoter operably linked to a tetracycline response element sequence;
[7] The method according to [5] or [6], wherein the step of inducing the expression of the AAV Cap protein is a step of contacting the cell with doxycycline or tetracycline;
[8] The method according to [5], wherein step (b) is carried out 6 to 36 hours after the start of step (a);
[9] The method according to [5], wherein step (b) is carried out 8 to 24 hours after the start of step (a);
[10] A nucleic acid construct comprising, in a direction from 5' to 3', a region containing a plurality of YY1-binding sequences, an AAV Rep protein-coding sequence, and an AAV Cap protein-coding sequence;
[11] The nucleic acid construct according to [10], wherein the YY1-binding sequence is a sequence selected from the group consisting of SEQ ID NO: 1 and SEQ ID NO: 2;
[12] The nucleic acid construct according to [10], wherein the YY1-binding sequence is a sequence of SEQ ID NO: 3;
[13] The nucleic acid construct according to [10], wherein the region containing a plurality of YY1-binding sequences is a sequence selected from the group consisting of SEQ ID NO: 4 and SEQ ID NO: 5;
[14] A cell comprising the nucleic acid construct according to any one of [10] to [13]; and
[15] a method for producing a recombinant AAV vector, the method comprising culturing the cell according to [14] to produce the recombinant AAV vector.

### EFFECTS OF THE INVENTION

The present invention provides a production method for obtaining an AAV vector with an increased AAV vector production amount (titer) and an increased proportion of mature AAV vectors. Further, provided are the nucleic acid constructs and the cells for use in the production method.

### BRIEF DESCRIPTION OF THE DRAWINGS

[Fig. 1] Fig. 1 shows the structures of plasmids prepared in Example 1.
[Fig. 2] Fig. 2 shows the titers, protein expression patterns, and infectivity of the AAV vectors prepared in Example 1.
[Fig. 3] Fig. 3 shows the amounts of Cap proteins in the virus solutions with the same titer, prepared in Example 1.
[Fig. 4] Fig. 4 shows the effect of separate expression of Rep gene and Cap gene on the AAV vector production in Example 2.
[Fig. 5] Fig. 5 shows the effect of Tet-Cap system on the AAV vector production amount in Example 2.
[Fig. 6] Fig. 6 shows the effect of Tet-Cap system on the proportion of mature AAV in Example 2.
[Fig. 7] Fig. 7 shows the effect of Tet-Cap system on the production of AAV vectors of other serotypes in Example 2.
[Fig. 8] Fig. 8 shows the effect of changes in Rep expression patterns on the AAV vector production in Example 2.
[Fig. 9] Fig. 9 shows the effect of adjusting the timing of Cap expression on the AAV vector production in Example 2.
[Fig. 10] Fig. 10 shows the effect of Tet-Cap system on the organ tropism of AAV vectors produced in Example 2.

### MODE FOR CARRYING OUT THE INVENTION

The present invention will be described in detail below.

### <Definition>

As used herein, the term "non-enveloped virus" refers to any virus other than enveloped viruses. The enveloped virus refers to any virus having a lipid layer or lipid bilayer on the viral surface. Representative examples of non-enveloped virus include, regarding viruses whose genome is DNA, adenovirus, parvovirus, papovavirus, and human papillomavirus, and regarding viruses whose genome is RNA, rotavirus, coxsackievirus, enterovirus, sapovirus, norovirus, poliovirus, echovirus, hepatitis A virus, hepatitis E virus, rhinovirus, and astrovirus.

The non-enveloped virus to which the production method of the present invention is applicable is not particularly limited. The production method of the present invention may be applied to a non-enveloped virus whose production method is already known, a non-enveloped virus newly obtained from nature, or a recombinant viral vector derived from any non-enveloped virus as described above. Preferable examples of the non-enveloped virus to be produced by the production method of the present invention include adenoviruses, and AAV and bocaviruses of the Parvoviridae family.

As used herein, AAV refers to a small virus belonging to the genus Dependovirus of the family Parvoviridae and capable of infecting primates including human and other vertebrates. AAV has a non-enveloped shell (capsid) of a regular icosahedron and contains a single strand of DNA inside the shell. As used herein, AAV includes the wild-type virus and derivatives thereof, and includes all serotypes and clades of AAV unless specified otherwise.

The present invention is applicable to AAV of all known serotypes, and may be used for, for example, at least one type of AAV selected from the group consisting of AAV1, AAV2, AAV3, AAV4, AAV5, AAV6, AAV7, AAV8, AAV9, AAVrh10, AAV-PHP.eB, and AAV-PHP.S. As used herein, the serotype of a recombinant AAV (rAAV) is based on the serotype from which the capsid is derived. In other words, the serotype of a rAAV is determined based on the source of cap gene used for preparation of rAAV, and does not depend on the serotype of an AAV genome encapsulated in the rAAV particle. For example, when the capsid is derived from AAV6 and ITRs in the AAV genome encapsulated in the rAAV particle are derived from AAV2, the rAAV is defined as serotype 6. Furthermore, the present invention may be applied to the production of AAV containing mutants of capsids of the above-mentioned AAV serotypes.

As used herein, the term "AAV vector" means an AAV having a vector function. That is, the AAV vector refers to an infectious particle that contains the AAV genome within the AAV particle. The term "AAV vector" includes an "AAV complete particle," an "AAV full particle," an "AAV vector particle," and an "AAV virion."

As used herein, the term "capsid" is one of components constituting a virus particle, and means a coat or shell consisting of a plurality of unit proteins (capsomeres) surrounding a genomic DNA or a core. A particle that is composed only of capsid proteins and does not contain any viral nucleic acid, core and other substances inside the capsid is called an "empty particle" or, sometimes, an "empty capsid particle", as used herein.

The present invention will be described in detail below.

### (1) Nucleic acid construct of the present invention

The present invention provides a nucleic acid construct comprising an inducible promoter and an adeno-associated virus (AAV) Cap protein-coding sequence operably linked to the promoter.

As used herein, the term "inducible promoter" refers to a promoter that can control the expression of a gene operably linked to the promoter depending on the presence or absence of an exogenous inducer or an exogenous stimulus. Herein, the term "operably linked" means a state such that the expression of a gene is controlled by the function of a control region (such as a promoter, etc.) as a result of the fact that the control region and a coding region of the gene are arranged in an appropriate positional relationship. The control of expression of a gene is a concept including the induction, enhancement, suppression, inhibition, etc. of transcription from the gene.

Types of the inducible promoter and the inducer or inducing stimulus may be appropriately selected by considering the type of a cell into which the promoter is introduced, the effect on proliferation of the cell, toxicity to the cell, ease of handling, and the like. Examples of the inducible promoter include, but not limited to, lactose-inducible promoters (lac promoter, lacUV5 promoter, tac promoter, trc promoter, Pspac promoter, etc.), galactose-inducible promoters (gall promoter, gal4 promoter, gal10 promoter, mel1 promoter, etc.), xylose-inducible promoters (xylA promoter, xylB promoter, etc.), arabinose-inducible promoters (araBAD promoter, etc.), rhamnose-inducible promoters (rhaBAD promoter, etc.), tetracycline-inducible promoters (tet promoter, etc.), hormone-inducible promoters (MMTV promoter, etc.), metal-inducible promoters (metallothionein promoter, etc.), alcohol-inducible promoters (alcA promoter, etc.), temperature-inducible promoters (λpL promoter, λpR promoter, etc.), heat shock-inducible promoters (hsp70 promoter, etc.), and light-inducible promoters (rbcS promoter). Preferred examples of the inducible promoter include, from the viewpoint of ease of handling, a promoter capable of inducing the expression of a gene linked to the promoter by addition of an inducer, and preferably a promoter capable of inducing the expression of a gene linked to the promoter in a dose-dependent manner in the presence of an inducer.

A preferred embodiment of the present invention is a nucleic acid construct comprising a tetracycline-inducible promoter. The tetracycline-inducible promoter is a promoter operably linked to a tetracycline response element sequence, and the function of the promoter is repressed by binding of a tetracycline (Tet) repressor to the tetracycline response element sequence. In the presence of the inducer tetracycline or a derivative thereof, the promoter induces the expression of a gene linked to the promoter, because the inducer or a derivative thereof binds to the Tet repressor to prevent the Tet repressor from binding to the tetracycline responsive element sequence. A tetracycline-inducible promoter comprising a combination of any constitutive promoter with a tetracycline response element sequence may be used in the present invention. Examples of the constitutive promoter include a CMV promoter, a β-actin promoter, a phosphoglycerate kinase promoter, an EF-1α promoter, and a CAG promoter. A preferable example of the tetracycline-inducible promoter, but not limited to, is a promoter known as Tet-On (registered trademark) system (composed of a tetracycline responsive element sequence and a CMV promoter). Examples of tetracycline or a derivative thereof include tetracycline, doxycycline, chlortetracycline, and oxytetracycline. From the viewpoint of the strength of inducing activity, doxycycline is preferred.

In the present invention, the AAV Cap protein-coding sequence expresses three types of capsid proteins (VP1, VP2, VP3) that assemble to form a viral capsid and an assembly-activating protein (AAP). As used herein, all of VP1, VP2 and VP3 are included in the capsid protein.

The nucleic acid construct of the invention may further comprise a nucleic acid construct comprising an AAV Rep protein-coding sequence which is expressed independently of the AAV Cap proteins. Herein, the nucleic acid construct of the present invention may be a single nucleic acid construct comprising the Cap protein-coding sequence and the Rep protein-coding sequence, or may be a combination of two nucleic acid constructs wherein the Cap protein-coding sequence and the Rep protein-coding sequence are contained in separate nucleic acid constructs. The Cap protein-coding sequence and the Rep protein-coding sequence may be derived from any AAV serotype. Furthermore, the Cap protein-coding sequence and the Rep protein-coding sequence may be derived from the same serotype or from different serotypes. It is preferable that the Rep protein-coding sequence is operably linked to a constitutive promoter.

In the nucleic acid construct of the present invention, a suitable control sequence other than the inducible promoter may be operably linked with the Cap protein-coding sequence. Examples of such a control sequence include a polyadenylation signal, a transcription termination sequence, an upstream regulatory domain, an origin of replication, an internal ribosome entry site (IRES), and an enhancer. The control sequence may be inherent in or foreign to the AAV from which the Cap protein is derived or may be a natural or synthetic sequence.

The nucleic acid construct of the present invention may be carried by a DNA to be used as a vector. Examples of such a DNA include a plasmid DNA, a phage DNA, transposon, a cosmid DNA, an episomal DNA, and a viral genome. The DNA can be particularly used to introduce the nucleic acid construct of the present invention into animal cells, preferably mammalian cells. The DNA is useful for introducing the nucleic acid construct of the present invention into cells in vitro, ex vivo and in vivo in order to confer the ability to express the AAV Cap proteins to the cells. Furthermore, the cells into which the nucleic acid construct of the present invention has been introduced are useful for producing AAV vectors.

### (2) Cells of the present invention

The present invention also provides a cell containing the nucleic acid construct of the present invention as above described in (1). The cell of the present invention is a cell that can be used for the production of an AAV vector, and is a cell that contains a nucleic acid construct comprising an AAV Cap protein-coding sequence operably linked to an inducible promoter. For example, the cell is a cell line maintained in vitro. The cell is useful for producing an AAV vector, as described below. The nucleic acid construct may be integrated into the genome of the cell of the present invention, or may be maintained within the cell so as to constitutively or transiently express the AAV capsid proteins.

A variety of cells, for example mammalian cells including mouse cells and primate cells (e.g., human cells), and insect cells, can be used as a host cell for producing the cell of the present invention. Suitable examples of the mammalian cells include, but not limited to, primary cells and cell lines. Suitable examples of the cell lines include HEK293 cells, 293EB cells, COS cells, HeLa cells, Vero cells, 3T3 mouse fibroblasts, C3H10T1/2 fibroblasts, CHO cells, and cells derived from these cells. Suitable examples of the insect cells include, but not limited to, primary cells and cell lines. Suitable examples of the cell lines include Sf9 cells and cells derived from them.

Introduction of the nucleic acid construct of the present invention into a host cell can be carried out by a known method. Examples of the known method include electroporation, calcium phosphate precipitation, direct microinjection into cells, liposome-mediated gene transfer, and nucleic acid delivery using high-velocity particle bombardment. When a viral vector carrying the nucleic acid construct of the present invention is used, an infection method suitable for the vector may be selected. Using such established techniques, the nucleic acid construct of the present invention is introduced stably into the chromosomes of a host cell or transiently into the cytoplasm of a host cell. For stable transformation, a well-known selection marker, for example, a neomycin resistance gene (encoding neomycin phosphotransferase) or a hygromycin B resistance gene [encoding aminoglycoside phosphotransferase (APH)] may be ligated to the nucleic acid construct of the present invention.

The cell of the present invention may comprise a nucleic acid construct comprising an AAV Rep protein-coding sequence which is expressed independently of the AAV Cap proteins. The Cap protein-coding sequence and the Rep protein-coding sequence may be contained in the same nucleic acid construct, or may be contained separately in two different nucleic acid constructs and the cell may comprise a combination of the two nucleic acid constructs. The Cap protein-coding sequence and the Rep protein-coding sequence may be derived from any AAV serotype. Furthermore, the Cap protein-coding sequence and the Rep protein-coding sequence may be derived from the same serotype or from different serotypes. It is preferable that the Rep protein-coding sequence is operably linked to a constitutive promoter.

Into the cell of the present invention, a "nucleic acid construct encoding an adenovirus-derived element necessary for AAV vector formation" and a "nucleic acid construct comprising an ITR sequence of AAV genomic DNA" may further be introduced. The cell of the present invention into which the above-described two nucleic acid constructs as well as the nucleic acid construct comprising the AAV Cap protein-coding sequence and the nucleic acid construct comprising the AAV Rep protein-coding sequence or the nucleic acid construct comprising the AAV Cap protein-coding sequence and the AAV Rep protein-coding sequence have been introduced can be used as an AAV producer cell (sometimes referred to as the AAV producer cell of the present invention).

Examples of the "adenovirus-derived element necessary for AAV vector formation" include, but not limited to, adenovirus-derived E1a protein, E1b protein, E2 protein, E4 protein, and VA RNA. These elements can be introduced into any cell to prepare a rAAV producer cell. Alternatively, a cell transiently or constitutively expressing one or some of the adenovirus-derived proteins necessary for AAV vector formation may be used as the host cell. The form of the "nucleic acid construct encoding an adenovirus-derived element necessary for AAV vector formation" is not limited. One or more nucleic acid constructs capable of supplying the adenovirus-derived elements may be introduced into a cell via a plasmid(s) or a viral vector(s) carrying the elements. The introduction of these nucleic acids into a cell can be carried out, for example, by a known method using commercially available or a known plasmid or viral vector. An example of such a plasmid is pHelper Vector (manufactured by TAKARA BIO INC.) . In place of the plasmid or viral vector, a virus having helper function, for example, adenovirus may be directly infected into a cell to achieve the above-described object.

The "nucleic acid construct comprising an ITR sequence of AAV genomic DNA" is composed of an AAV-derived ITR sequence and a nucleic acid desired to be carried in an AAV vector. Examples of the nucleic acid desired to be carried in an AAV vector include nucleic acids that supply any foreign gene, for example, polypeptides (enzymes, growth factors, cytokines, receptors, structural proteins, etc.), antisense RNA, ribozymes, decoys, RNA that causes RNA interference, etc. In addition, to control the expression of the foreign gene, at least one selected from the group consisting of a suitable promoter, enhancer, terminator and other transcriptional regulatory elements may be inserted into the nucleic acid to be encapsulated in the AAV vector. The nucleic acid to be encapsulated in the AAV vector can be introduced into a cell as a nucleic acid construct in the form of a plasmid. The plasmid as described above can be constructed, for example, by a known method using a commercially available or known plasmid. An example of such a plasmid is pAAV-EGFP.

### (3) Method for producing recombinant AAV vector of the present invention

The present invention also provides a method for producing a recombinant AAV vector. More specifically, one aspect of the present invention is a method for producing a recombinant AAV vector, the method comprising:
(a) culturing the AAV producer cell of the present invention;
(b) inducing the expression of the AAV Cap protein in the cell of step (a); and
(c) culturing the cell of step (b) to produce a recombinant AAV vector.

### <Step (a): Step of culturing AAV producer cell of the present invention>

Step (a) is a step of culturing an AAV producer cell. The AAV producer cell is the cell of the present invention as described above in (2), comprising the nucleic acid construct of the present invention and the nucleic acid construct necessary for producing an AAV vector. A medium used for culturing the cell may contain components necessary for cell culture. Examples of such a medium include basic synthetic media, such as MEM, DMEM, IMDM, DMEM:F-12, RPMI1640, Ham's F-12, and EMEM (all commercially available from Thermo Fisher, etc.), as well as these basic synthetic media supplemented with fetal bovine serum, growth factors, peptides, and/or increased amounts of amino acids, as necessary.

The AAV producer cell can be cultured under known culture conditions depending on the type of cell. For example, the culture may be performed at a temperature of 30 to 37°C, a humidity of 95%, and a CO₂ concentration of 5 to 10%, though the present invention is not limited to these conditions. The culture may be carried out at temperature, humidity, and CO₂ concentration outside the above-mentioned ranges as long as proliferation of the desired cell is attained.

Examples of cell culture equipment (containers) that can be used include a petri dish, a flask, a bag, a large culture tank, and a bioreactor. As the bag, a CO₂ gas-permeable bag for cell culture is preferred. When a large number of cells are required, a large culture tank may be used.

### <Step (b): Step of inducing expression of AAV Cap protein in cell of step (a)>

Step (b) is a step of inducing the expression of AAV Cap protein in the cell of step (a). This step is carried out by allowing the inducible promoter operably linked to the Cap protein-coding sequence to function. Thus, in this step, an appropriate operation may be selected depending on the type of the promoter operably linked to the Cap protein-coding sequence. This step is usually performed by altering the culture conditions (temperature etc.) or by contacting the cell with the inducer. When the inducer is used, the induction of Cap protein expression is achieved by replacing the medium in step (a) with a medium containing the inducer or by adding the inducer to the medium in step (a).

The concentration of the inducer in the medium used in the present invention is not particularly limited and may be such concentration that the inducer acts as an active ingredient. In the case of doxycycline, for example, the concentration is in the range of about 0.01 ug/ml to about 30 mg/ml. Preferably, the doxycycline concentration is from about 0.05 mg/ml to about 1 mg/ml, more preferably from about 0.05 mg/ml to about 10 mg/ml, or most preferably from about 1 mg/ml to about 20 mg/ml.

The timing of carrying out step (b) is not particularly limited. Step (b) may be carried out immediately after the initiation of step (a) or may be preferably carried out after the AAV producer cell is proliferated to a certain extent. For example, step (b) may be carried out 3 to 48 hours after the initiation of step (a), preferably 6 to 36 hours after the initiation of step (a), and more preferably 8 to 24 hours after the initiation of step (a).

For example, when the promoter operably linked to the AAV Cap protein-coding sequence is a promoter to which a tetracycline response element sequence is operably linked, tetracycline or a derivative thereof is used as the inducer in step (b). Examples of the tetracycline or derivative thereof include tetracycline, doxycycline, chlortetracycline, and oxytetracyclin. Preferred is doxycycline or tetracycline.

### <Step (c):Step of culturing cell of step (b) to produce recombinant AAV vector>

Step (c) is a step of culturing the cell of step (b) to produce a recombinant AAV vector. This step is carried out by culturing the cell under the induction of the AAV Cap protein expression in step (b). Culturing the cell of step (b) under conditions in which the promoter functions, for example, in the presence of the inducer can result in induction of the AAV vector production, and the AAV vector can be consequently obtained.

A culture period in step (c) is not particularly limited, and may be, for example, 24 hours to 14 days, preferably 2 to 7 days. Understandably, the culture period is appropriately determined depending on the concentration of the inducer. Furthermore, culture conditions such as temperature, humidity, and CO₂ concentration may be appropriately determined and are not particularly limited. For example, the culture conditions may be the same as those in step (a). A culture medium is not particularly limited as long as it contains components necessary for cell culture. For example, the culture medium may be the same medium as in step (a). When the inducer is used in step (b), the medium may contain a suitable inducer at an appropriate concentration. Furthermore, the culture conditions, for example the concentration of the inducer, may be appropriately changed during the culture period.

During the culture, the AAV vector is produced within the cell and/or in a culture supernatant. The AAV vectors obtained by this step have characteristics including a high titer and a high proportion of mature AAV vectors. The term "titer" refers to the amount of infectious viruses contained in a sample. The titer of an AAV vector is an index of the amount of AAV vectors produced, and can be determined, for example, by measuring the number of AAV vector genomes in a virus solution by PCR or the like, or by measuring the ability to infect cells. The mature AAV vector refers to an AAV vector having an AAV genome, and does not include AAV particles having no AAV genome (empty particles) nor incomplete AAV particles. The proportion of mature AAV vectors in the produced AAV vectors can be determined, for example, by detecting intact virus particles in a virus solution using an immunological technique and determining a ratio between the detected amount and the number of vector genomes (genome titer). The term "mature vector" includes a "complete particle," a "full particle," a "vector particle," and a "virion."

The AAV vector produced by the method of the present invention can be purified from the AAV producer cell culture (cells and culture supernatant) by a known method. Disruption or lysis of cells can be carried out by a known method, for example, ultrasonication, freeze-thaw treatment, enzyme treatment, or osmotic pressure treatment. For example, a surfactant may be added to a culture containing the AAV producer cells to lyse the cells. The surfactant is not particularly limited, and an example of the surfactant is Triton X-100. The concentration of the surfactant is not particularly limited, and examples of the concentration include a final concentration of 0.01 to 1%, preferably a final concentration of 0.05 to 0.5%, and more preferably a final concentration of 0.08 to 0.12%. The temperature and time during contact with the surfactant are not particularly limited, and examples of the temperature include 0 to 40°C, and preferably 4 to 37°C, and examples of the time include 5 minutes to 8 hours, and preferably 10 minutes to 4 hours, and more preferably 30 minutes to 2 hours. A crude extract can be obtained by centrifuging the culture containing the lysed cells and collecting a supernatant.

In the method of the present invention, the crude extract can then be subjected to treatment with nuclease, and further subjected to purification by ultracentrifugation, chromatography, ultrafiltration, precipitant treatment, or other known methods to obtain a concentrated or purified AAV vector as the final product. For purification, a commercially available reagent or kit can be used. For example, AAVpro (registered trademark) Purification Kit (manufactured by Takara Bio Inc.) may be used.

The AAV vector obtained by the method of the present invention can be stored in an appropriate solution for a long period of time. For example, the rAAV vector can be stored in a phosphate buffered saline (pH 7.4), at -80°C, for example, for 12 hours or more, 1 day or more, 3 days or more, 1 week or more, 2 weeks or more, 1 month or more, 2 months or more, 3 months or more, or 6 months or more.

### (4) Nucleic acid construct comprising YY1-binding sequence of the present invention

Furthermore, the present invention provides a nucleic acid construct comprising, in order from the 5' end to the 3' end, a region comprising multiple YY1-binding sequences, an AAV Rep protein-coding sequence, and an AAV Cap protein-coding sequence.

YY1 (Yin Yang 1) is, in the case of human, a transcription factor encoded by a YY1 gene. YY1 is a transcription factor distributed ubiquitously in the living body and belongs to GLI-Kruppel zinc finger protein. YY1 is known to bind to YY1-binding sequences and is involved in repression and activation of a variety of promoters.

The YY1 binding sequence used in the present invention is not particularly limited as long as the effects of the present invention of obtaining an AAV vector having a high titer and a high maturation rate are produced. Examples of the YY1 binding sequence include ACATNTT (SEQ ID NO: 1) or CCATNTT (SEQ ID NO: 2) wherein N is any base (N=A, C, G, or T), and sequences comprising the above-mentioned sequences. Within these YY1-binding sequences, the first A or C, the second C, the third A, and the fourth T are essential for binding to YY1. The YY1 binding sequence used in the present invention is preferably CTCCATTTT (SEQ ID NO: 3) .

In the nucleic acid construct of the present invention, multiple YY1 binding sequences exist. The number of YY1 binding sequences contained in the nucleic acid construct is not particularly limited. For example, two or more, and preferably four YY1 binding sequences may exist. These multiple YY1-binding sequences may have different nucleotide sequences as long as they exert their function. Other nucleotide sequences may be sandwiched between the multiple YY1-binding sequences. The region containing multiple YY1 binding sequences is preferably CTCCATTTTGAGTCTCCATTTT (SEQ ID NO: 4) or CTCCATTTTGAGTCTCCATTTTGAGTCTCCATTTTGAGTCTCCATTTT (SEQ ID NO: 5) .

The nucleic acid construct of the present invention comprising YY1-binding sequences comprises a promoter for expressing the Rep proteins and the Cap proteins. For example, the YY1 binding sequences are placed between the promoter and the sequence encoding Rep and Cap proteins. Examples of the promoter that can be used include, but not limited to, promoters of wild-type AAV that contribute to the expression of Rep proteins and Cap proteins, and heterologous promoters, for example, virus-derived promoters (CMV promoter, MSCV promoter, SV40 promoter, etc.) and mammalian-derived promoters (β-actin promoter, phosphoglycerate kinase promoter, EF-1α promoter, etc.). The nucleic acid construct of the present invention may further comprise a regulatory sequence, for example, a polyadenylation signal, a transcription termination sequence, an enhancer sequence, or the like.

The nucleic acid construct of the present invention comprising the YY1-binding sequences can be used by being carried in an appropriate vector. The vector may be selected depending on the intended use of the nucleic acid construct, and is not particularly limited. The vector may be a vector capable of self-replication in a host cell or a vector that can be integrated into host chromosomes. Examples of the vector include a plasmid vector, a phage vector, a viral vector, and a transposon.

The nucleic acid construct of the present invention comprising the YY1 binding sequences may be used to produce AAV vectors. A cell into which the nucleic acid construct is introduced expresses the Rep proteins and Cap proteins. Thus, the cell acquires the ability to produce an AAV vector, when the "nucleic acid construct encoding an adenovirus-derived element necessary for AAV vector formation" and the "nucleic acid construct comprising an ITR sequence of AAV genomic DNA" are further introduced into the cell. The AAV vector producer cell thus prepared can be cultured to produce an AAV vector in the culture. The culture conditions are not particularly limited. The AAV vector producer cell may be cultured in a medium suitable for the host cell, for example, under the conditions of a temperature of 30 to 37°C and a CO₂ concentration of 5 to 10%. The AAV vector in the culture can be purified or concentrated by a known method.

The AAV vectors thus produced are characterized by a high titer and a high proportion of mature AAV vectors, etc.

### EXAMPLES

The present invention will be more specifically explained with reference to Examples described below, which the scope of the present invention is not limited to.

### Example 1: Improvement of AAV vector production by modification located upstream of RepCap gene in pAAV-RC2

### (Method)

### 1-1. Insertion of YY1 binding sequence etc. into the upstream of RepCap gene in pAAV-RC2

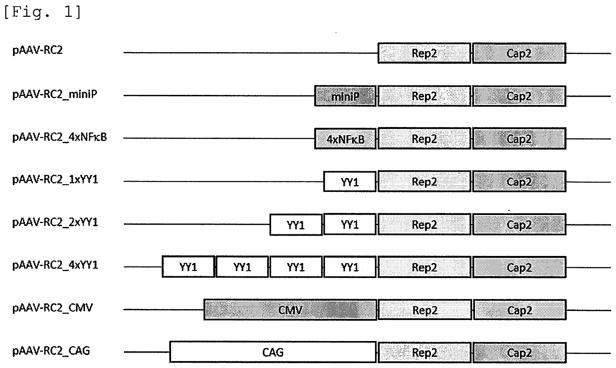

To insert a 1 x YY1 binding sequence into the upstream of RepCap genes in pAAV-RC2 (Agilent), inverse PCR was performed using the plasmid as a template and primers shown by SEQ ID NO: 6 and SEQ ID NO: 7. Then, an amplified DNA fragment was self-ligated using T4 DNA ligase to form a circular plasmid. Escherichia coli was transformed with the circular plasmid. Then, clone selection was performed, and the plasmid containing the inserted sequence of interest was determined by DNA sequencing (pAAV-RC2_1xYY1). For insertion of a 2 x YY1 binding sequence, oligonucleotides of SEQ ID NO: 8 and SEQ ID NO: 9 were prepared and used to form a double strand. The termini of the double-stranded oligonucleotide were phosphorylated with T4 polynucleotide kinase and cloned into a HpaI-XbaI site in pAAV-RC2 (pAAV-RC2_2xYY1). For insertion of a 4 x YY1 binding sequence, oligonucleotides of SEQ ID NO: 10 and SEQ ID NO: 11 were prepared and used to form a double strand. The termini of the double-stranded oligonucleotide were phosphorylated with T4 polynucleotide kinase and cloned into a HpaI-XbaI site in pAAV-RC2 (pAAV-RC2_4xYY1). Furthermore, a 4xNF-κB sequence, a minimal promoter (miniP), a CMV promoter, or a CAG promoter was inserted into the upstream of the RepCap genes in pAAV-RC2 to prepare plasmids (pAAV-RC2_4xNF-κB, pAAV-RC2_miniP, pAAV-RC2_CMV, pAAV-RC2_CAG). The structures of the prepared plasmids are shown in FIG. 1.

### 1-2. Production of AAV vectors

HEK293 cells were transfected with each plasmid prepared in 1-1 or pAAV-RC2, pHelper (TAKARA BIO INC.), and pAAV-EGFP which is an AAV vector plasmid containing an EGFP expression cassette in a ratio of 1:1:1 (pAAV-RC2 = 1 µg, pHelper = 1 µg, pAAV-EGFP = 1 µg) using HilyMax Transfection Reagent (DOJINDO LABORATORIES). After the transfected cells were cultured in a medium [MEM (WAKO) containing 10% FBS (Gibco), 1x non-essential amino acids (NEAA) (WAKO) and antibiotics (Gibco)] for 12 hours, the medium was replaced with the fresh medium. The cells and a supernatant were collected together 72 hours after the medium replacement, and frozen at -80°C. The frozen cell/supernatant sample was thawed at 37°C, vortexed for 30 seconds, and then flash frozen in dry ice-ethanol. After such a freeze-thaw process was repeated a total of four times, the sample was centrifuged and a supernatant was collected. The supernatant was passed through a 0.45 um filter to remove impurities, and the resulting solution was dispensed as a virus solution and stored.

### 1-3. Measurement of AAV titer by qPCR

To measure the titer of AAV vectors contained in the virus solutions obtained in 1-2, qPCR was performed using primers shown by SEQ ID NO: 12 and SEQ ID NO: 13 (HUMAN GENE THERAPY METHODS: Part B 23:18-28) and TB green Premix Ex Taq (TAKARA BIO INC.). As a standard, serial dilutions of 10⁷, 10⁶, 10⁵, 10⁴, 10³ and 10² were prepared using AAV with a known titer. The dilutions and each virus solution were treated at 96°C for 10 minutes before they were subjected to each qPCR. Then, the AAV titer of the virus solution (the number of vector genomes in the solution = vg/µL) was calculated from the qPCR data.

### 1-4. Confirmation of AAV vector infectivity

The virus solutions obtained in 1-2 were adjusted to have the same titer based on the qPCR data, and then used to infect 2V6.11 cells seeded in 96-well plates. After the infected cells were cultured for 3 to 5 days, the cells were observed by immunofluorescence or confocal microscopy.

### 1-5. Detection of Rep protein and Cap protein by Western blot

HEK293 cells were transfected with various plasmids under the same conditions as described in 1-2. After the transfected cells were cultured in a medium [MEM containing 10% FBS, 1x non-essential amino acids (NEAA) and antibiotics] for 12 hours, the medium was replaced with the fresh medium. The cells were washed with a phosphate-buffered saline (PBS) 72 hours after the medium replacement, collected, and lysed in a 1×SDS sample buffer. Then, the sample was treated at 99°C for 10 minutes, and subjected to SDS-PAGE and Western blotting. For detection of Rep proteins, an anti-Rep mouse monoclonal 303.9 antibody (Progen) was used as the primary antibody. For detection of Cap proteins, an anti-Cap rabbit polyclonal antibody (Progen) was used as the primary antibody. As the secondary antibody, an anti-mouse IgG-HRP (Cell signaling technology) or an anti-rabbit IgG-HRP (Cell signaling technology) was used.

### 1-6. Measurement of Cap protein by immunoprecipitation

Anti-intact particle AAV Ab (A20 for AAV2) and Protein A/B magnetic beads (Dynabeads) were reacted to prepare antibody-bead complexes. The antibody-bead complexes were washed using a magnetic stand, and resuspended in an IP buffer (cell lysis buffer). Then, the antibody-bead complexes were added to each of the virus solutions that were adjusted to have the same titer based on the qPCR data. After reaction at room temperature for 1 hour, AAV-antibody-bead complexes were collected using a magnetic stand, washed 3 to 4 times, and dissolved in a 1×SDS sample buffer. Then, the sample was treated at 99°C for 10 minutes and subjected to SDS-PAGE and Western blotting. For detection of Cap proteins, an anti-Cap rabbit polyclonal antibody or an anti-Cap mouse monoclonal B1 antibody (Progen) was used as the primary antibody, and an anti-rabbit IgG-HRP (Cell signaling technology) or a Clean-blot IP detection reagent (ThermoFisher) was used as the secondary antibody.

### (Results)

### 1. Improvement of AAV vector production by modifying the upstream of RepCap gene in pAAV-RC2

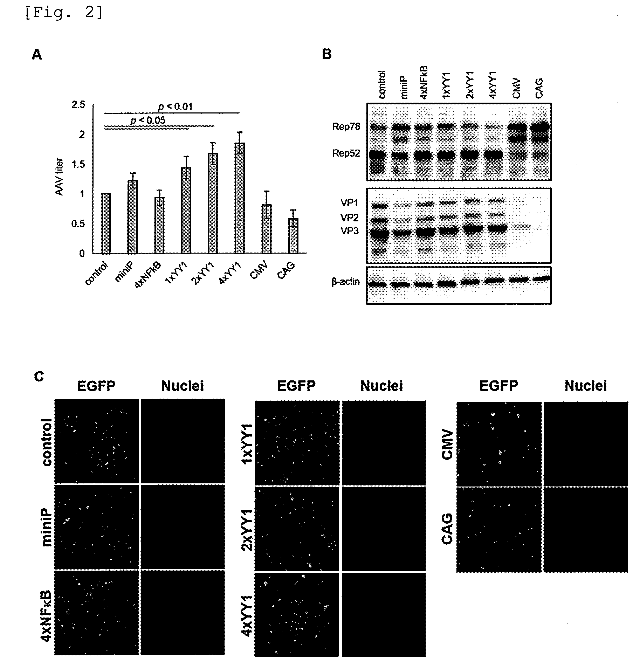

In some plasmids containing RepCap genes, a part of a promoter region derived from the AAV genome may remain at the upstream of the RepCap genes. When such plasmids are used to produce AAV vectors, the production of AAV vectors may be improved as compared to the AAV vector production when a control plasmid is used. Based on this fact, there is a possibility that the production of AAV vectors is improved by the presence of modification located at the upstream of RepCap genes. Thus, various sequences (miniP, 4xNF-κB, 1xYY1, 2xYY1, 4xYY1, CMV, CAG) were inserted at the upstream of the RepCap genes in pAAV-RC2 (Fig. 1). The plasmids thus obtained were used to produce AAV vectors. The AAV titers (the amount of AAV vectors produced) of the obtained AAV vectors were measured (Fig. 2A), and the expression patterns of Rep proteins and Cap proteins in the AAV vectors were confirmed (Fig. 2B).

When the CMV promoter or CAG promoter was inserted into pAAV-RC2 and the plasmid was used to produce AAV vectors, the amount of AAV vectors produced was almost the same as that of when pAAV-RC2 (control) was used. Regarding differences in the expression patterns of Rep proteins and Cap proteins, when the CMV promoter or CAG promoter was inserted into pAAV-RC2 and the plasmid was used to produce AAV vectors, the AAV vectors produced had an increased expression level of Rep78 and decreased expression levels of Cap proteins as compared to those of when pAAV-RC2 (control) was used. The insertion of the 4xNF-κB sequence did not result in an increase in the production amount of AAV vector and any significant difference in the expression patterns of Rep and Cap proteins. The insertion of miniP increased the production amount of AAV vector by about 1.2 times. Regarding differences in the expression patterns of Rep proteins, the insertion of miniP resulted in a slight increase in the expression level of Rep78 and a slight decrease in the expression level of Rep52, as compared to the expression patters in AAV vectors produced when the control was used. On the other hand, the insertion of miniP resulted in slight decreases in the expression levels of Cap proteins. The insertion of the 1xYY1 sequence increased the production amount of AAV vector by about 1.4 times. The insertion of the 2xYY1 sequence or the 4xYY1 sequence increased the production amount of AAV vector by about 1.6 times or about 1.8 times, respectively. On the other hand, the insertion of the 1xYY1 sequence, 2xYY1 sequence or 4xYY1 sequence resulted in no significant differences in the expression patterns of Rep proteins and Cap proteins.

Furthermore, when 2V6.11 cells were infected with the virus solutions that were adjusted to have the same titer based on the qPCR values, no significant differences were observed in the infectivity of the AAV vectors produced using various plasmids (FIG. 2C).

### 2. Improvement of maturation rate of AAV vector by modifying the upstream of RepCap gene in pAAV-RC2

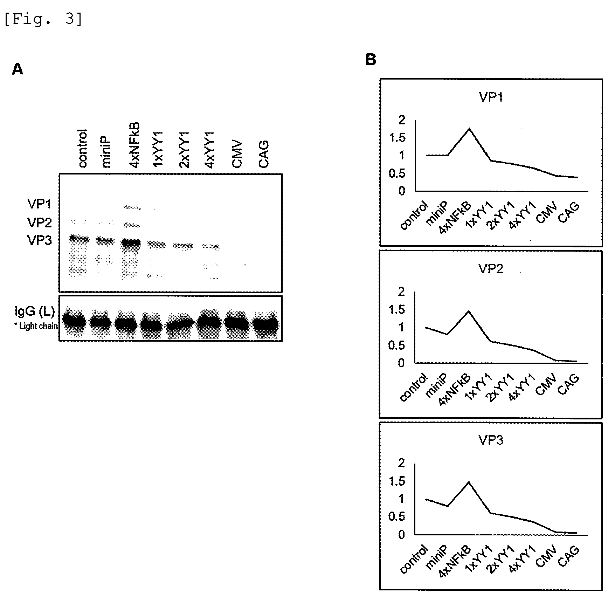

Furthermore, the virus solutions adjusted to have the same titer based on the qPCR values were subjected to immunoprecipitation using an antibody specific for intact particles (including empty particles), and then to Western blotting to measure Cap proteins (Fig. 3A and 3B). The insertion of miniP slightly decreased the amounts of Cap proteins. The insertion of the 4xNF-κB sequence increased the amounts of Cap proteins. The insertion of the 1xYY1, 2xYY1, or 4xYY1 sequence significantly decreased the amounts of Cap proteins. The insertion of the 4xYY1 sequence resulted in a particularly significant decrease in the amounts of Cap proteins. The insertion of the CMV promoter or the CAG promoter resulted in a noticeable decrease in the amounts of Cap proteins. Among the samples having the same titer, the samples containing lower amounts of Cap proteins probably contain a lower number of empty particles which do not contain viral genomes. Thus, it was found that the insertion of the YY1 binding sequence (s) into the upstream of the RepCap genes increased the proportion of mature AAV particles.

### Example 2: Improvement of production of mature AAV vector by adjustment of timing of Cap expression

### (Method)

### 2-1. Preparation of plasmid

PCR was performed using pAAV-RC2 as a template and primers shown by SEQ ID NO: 14 and SEQ ID NO: 15. As a polymerase, KOD-plus-Neo (TOYOBO) was used (hereinafter, the same polymerase was used). An amplified fragment (a Rep2 gene together with a part of the upstream sequence and a part of the downstream sequence; preRep2) was treated with NheI and BglII, and then inserted into a NheI-BamHI site of pcDNA3.1 (ThermoFisher) to obtain pCMV-Rep2.

PCR was performed using pAAV-RC2 as a template and primers shown by SEQ ID NO: 16 and SEQ ID NO: 17. An amplified fragment (a Cap2 gene together with a part of the upstream sequence and a part of the downstream sequence; preCap2) was treated with NheI and BamHI, and then inserted into a NheI-BamHI site of pcDNA3.1 to obtain pCMV-Cap2.

PCR was performed using pAAV-RC2 as a template and primers shown by SEQ ID NO: 18 and SEQ ID NO: 19. An amplified fragment (from the initiation codon to the termination codon of Rep52) was treated with NheI and XhoI, and then inserted into a XbaI-SalI site of pIRES (Clontech) to obtain pIRES-Rep52. Next, PCR was performed using pAAV-RC2 as a template and primers shown by SEQ ID NO: 20 and SEQ ID NO: 21. An amplified fragment (from the initiation codon to the termination codon of Rep78) was treated with NheI and XhoI, and then inserted into a NheI-XhoI site of pIRES-Rep52 to obtain pCMV-78-I-52.

PCR was performed using pAAV-RC2 as a template and primers shown by SEQ ID NO: 20 and SEQ ID NO: 21. An amplified fragment (from the initiation codon to the termination codon of Rep78) was treated with NheI and XhoI, and then inserted into a XbaI-SalI site of pIRES (Clontech) to obtain pIRES-Rep78. Next, PCR was performed using pAAV-RC2 as a template and primers shown by SEQ ID NO: 18 and SEQ ID NO: 19. An amplified fragment (from the initiation codon to the termination codon of Rep52) was treated with NheI and XhoI, and then inserted into a NheI-XhoI site of pIRES-Rep78 to obtain pCMV-52-I-78.

Oligonucleotides of SEQ ID NO: 22 and SEQ ID NO: 23 were annealed and their ends were phosphorylated with T4 polynucleotide kinase. This double-stranded oligonucleotide was then inserted into a EcoRV-EcoRI site of pBlueScript SK(+) (Agilent) to obtain pBlueScript-2A. Next, PCR was performed using pAAV-RC2 as a template and primers shown by SEQ ID NO: 18 and SEQ ID NO: 24. An amplified fragment (from the initiation codon to just before the termination codon of Rep52) was treated with XhoI and EcoRV, and then inserted into a XhoI-EcoRV site of pBlueScript-2A to obtain pBlueScript-Rep52-2A. Next, PCR was performed using pAAV-RC2 as a template and primers shown by SEQ ID NO: 20 and SEQ ID NO: 21. An amplified fragment (from the initiation codon to the termination codon of Rep78) was treated with MfeI and XbaI, and then inserted into a MfeI-XbaI site of pBlueScript-Rep52-2A to obtain pBlueScript-Rep52-2A-Rep78. Next, pBlueScript-Rep52-2A-Rep78 was treated with NheI and Xbal. A fragment thus obtained (Rep52-2A-Rep78) was inserted into a NheI-XbaI site of pcDNA3.1 to obtain pCMV-52-2A-78.

PCR was performed using pAAV-RC2 as a template and primers shown by SEQ ID NO: 16 and SEQ ID NO: 17. An amplified fragment (preCap2) was treated with NheI and BamHI, and then inserted into a NheI-BamHI site of pCW-MCS-WPRE-PGK-Hyg-P2A-rTetR comprising a Tet-dependent gene expression promoter and a Tet repressor to obtain pCW-preCap2-WPRE-PGK-Hyg-P2A-rTetR (pTet-Cap2).

As pAAV-RC5, pRC5 (TAKARA BIO INC.) was used. PCR was performed using pAAV-RC5 as a template and primers shown by SEQ ID NO: 16 and SEQ ID NO: 17. An amplified fragment (preCap5) was treated with NheI and EcoRV, and then inserted into a NheI-HpaI site of pCW-MCS-WPRE-PGK-Hyg-P2A-rTetR to obtain pCW-preCap5-WPRE-PGK-Hyg-P2A-rTetR (pTet-Cap5).

PCR was performed using pRepCap6 (Addgene) as a template and primers shown by SEQ ID NO: 25 and SEQ ID NO: 26. The 3' end of an amplified fragment (preCap6) was treated with XmaI, and the 5' end of the fragment was phosphorylated. Then, the fragment was inserted into a SwaI-XmaI site of pAAV-RC2 to obtain pAAV-RC6. PCR was performed using pAAV-RC6 as a template and primers shown by SEQ ID NO: 16 and SEQ ID NO: 27. An amplified fragment (preCap6) was treated with NheI and PmeI, and then inserted into a NheI-HpaI site of pCW-MCS-WPRE-PGK-Hyg-P2A-rTetR to obtain pCW-preCap6-WPRE-PGK-Hyg-P2A-rTetR (pTet-Cap6).

PCR was performed using pAAV2/1 (Addgene) as a template and primers shown by SEQ ID NO: 25 and SEQ ID NO: 28. The 3' end of an amplified fragment (preCap1) was treated with XmaI, and the 5' end of the fragment was phosphorylated. Then the fragment was inserted into a SwaI-XmaI site of pAAV-RC2 to obtain pAAV-RC1. PCR was performed using pAAV-RC1 as a template and primers shown by SEQ ID NO: 16 and SEQ ID NO: 29. An amplified fragment (preCap1) was treated with NheI and EcoRV, and then inserted into a NheI-HpaI site of pCW-MCS-WPRE-PGK-Hyg-P2A-rTetR to obtain pCW-preCap1-WPRE-PGK-Hyg-P2A-rTetR (pTet-Cap1).

After pAAV2/7 (Addgene) was treated with SwaI and PmeI, the resulting fragment (preCap7) was inserted into a SwaI-PmeI site of pAAV-RC2 to obtain pAAV-RC7. PCR was performed using pAAV2/7 as a template and primers shown by SEQ ID NO: 16 and SEQ ID NO: 30. An amplified fragment (preCap7) was treated with NheI and EcoRV, and then inserted into a NheI-HpaI site of pCW-MCS-WPRE-PGK-Hyg-P2A-rTetR to obtain pCW-preCap7-WPRE-PGK-Hyg-P2A-rTetR (pTet-Cap7).

After pAAV2/8 (Addgene) was treated with SwaI and PmeI, the resulting fragment (preCap8) was inserted into a SwaI-PmeI site of pAAV-RC2 to obtain pAAV-RC8. PCR was performed using pAAV2/8 as a template and primers shown by SEQ ID NO: 16 and SEQ ID NO: 30. An amplified fragment (preCap8) was treated with NheI and EcoRV, and then inserted into a NheI-HpaI site of pCW-MCS-WPRE-PGK-Hyg-P2A-rTetR to obtain pCW-preCap8-WPRE-PGK-Hyg-P2A-rTetR (pTet-Cap8).

After pAAV2/9n (Addgene) was treated with SwaI and PmeI, the resulting fragment (preCap9) was inserted into a SwaI-PmeI site of pAAV-RC2 to obtain pAAV-RC9. PCR was performed using pAAV2/9n as a template and primers shown by SEQ ID NO: 16 and SEQ ID NO: 30. An amplified fragment (preCap9) was treated with NheI and EcoRV, and then inserted into a NheI-HpaI site of pCW-MCS-WPRE-PGK-Hyg-P2A-rTetR to obtain pCW-preCap9-WPRE-PGK-Hyg-P2A-rTetR (pTet-Cap9).

After pAAV2/rh10 (Addgene) was treated with SwaI and PmeI, the resulting fragment (preCap rh10) was inserted into a SwaI-PmeI site of pAAV-RC2 to obtain pAAV-RC rh10. PCR was performed using pAAV2/rh10 as a template and primers shown by SEQ ID NO: 16 and SEQ ID NO: 30. An amplified fragment (preCap rh10) was treated with NheI and EcoRV, and then inserted into a NheI-HpaI site of pCW-MCS-WPRE-PGK-Hyg-P2A-rTetR to obtain pCW-preCap rh10-WPRE-PGK-Hyg-P2A-rTetR (pTet-Cap rh10).

PCR was performed using pUCmini-iCAP-PHP.eB (Addgene) as a template and primers shown by SEQ ID NO: 31 and SEQ ID NO: 32. An amplified fragment (preCap PHP.eB) was treated with SwaI and XmaI, and then inserted into a SwaI-XmaI site of pAAV-RC2 to obtain pAAV-RC PHP.eB. PCR was performed using pAAV-RC PHP.eB as a template and primers shown by SEQ ID NO: 16 and SEQ ID NO: 17. An amplified fragment (preCap PHP.eB) was treated with NheI and EcoRV, and then inserted into a NheI-HpaI site of pCW-MCS-WPRE-PGK-Hyg-P2A-rTetR to obtain pCW-preCap PHP.eB-WPRE-PGK-Hyg-P2A-rTetR (pTet-Cap PHP.eB).

PCR was performed using pUCmini-iCAP-PHP.S (Addgene) as a template and primers shown by SEQ ID NO: 31 and SEQ ID NO: 32. An amplified fragment (preCap PHP.S) was treated with SwaI and XmaI, and then inserted into a SwaI-XmaI site of pAAV-RC2 to obtain pAAV-RC PHP.S. PCR was performed using pAAV-RC PHP.S as a template and primers shown by SEQ ID NO: 16 and SEQ ID NO: 17. An amplified fragment (preCap PHP.S) was treated with NheI and EcoRV, and then inserted into a NheI-HpaI site of pCW-MCS-WPRE-PGK-Hyg-P2A-rTetR to obtain pCW-preCap PHP.S-WPRE-PGK-Hyg-P2A-rTetR (pTet-Cap PHP.S) .

After pCMV-Cap2 was treated with AgeI and EcoRV, the resulting fragment (preCap2) was inserted into an AgeI-EcoRV site of pcDNA4/TO (Invitrogen) to obtain pcDNA4/TO-preCap2.

### 2-2. Production of AAV vector by Tet-Cap system

To construct a system for producing AAV vectors while adjusting the timing of Cap expression (Tet-Cap system), HEK293 cells were transfected with pAAV-EGFP, pHelper, pTet-Cap2, and pCMV-Rep2 in a ratio of pAAV-EGFP : pHelper : pTet-Cap2 : pCMV-Rep2 = 1 : 1 : 1 : 0.25, using HilyMax Transfection Reagent (DOJINDO LABORATORIES). As a control, HEK293 cells were transfected with pAAV-EGFP, pHelper, pAAV-RC2, and pcDNA3.1 in a ratio of pAAV-EGFP : pHelper : pAAV-RC2 : pcDNA3.1 = 1 : 1 : 1 : 0.25. Twelve hours after transfection, a medium was replaced with a fresh medium [MEM containing 10% FBS, 1x non-essential amino acids (NEAA) and antibiotics] containing doxycycline at a final concentration of 2 µg/mL, and the cells were cultured for 72 hours after the medium replacement. The cells and a supernatant were collected together, and frozen at -80°C. The frozen cell/supernatant sample was thawed at 37°C, vortexed for 30 seconds, and then flash frozen in dry ice-ethanol. After such a freeze-thaw process was repeated a total of four times, the sample was centrifuged and a supernatant was collected. The supernatant was passed through a 0.45 um filter to remove impurities, and the resulting solution was dispensed as a virus solution and stored.

2-3. Measurement of AAV titer by qPCR

To measure the titer of the virus solutions obtained in 2-2, qPCR was performed using primers shown by SEQ ID NO: 12 and SEQ ID NO: 13 (HUMAN GENE THERAPY METHODS: Part B 23:18-28) and TB green Premix Ex Tag (TAKARA BIO INC.). As a standard, serial dilutions of 10⁷, 10⁶, 10⁵, 10⁴, 10³, and 10² were prepared using AAV with a known titer. The dilutions and each virus solution were treated at 96°C for 10 minutes before they were subjected to each qPCR. Then, the AAV titer of the virus solution (the number of vector genomes in the solution = vg/µL) was calculated from the qPCR data.

### 2-4. Confirmation of AAV vector infectivity

The virus solutions obtained in 2-2 were adjusted to have the same titer based on the qPCR data, and then used to infect 2V6.11 cells seeded in 96-well plates. After the infected cells were cultured for 3 to 5 days, the cells were observed by immunofluorescence or confocal microscopy.

### 2-5. Detection of Rep protein and Cap protein by Western blot

HEK293 cells were transfected with various plasmids under the same conditions as described in 2-2. After the transfected cells were cultured for 12 hours, a medium was replaced with a fresh medium [MEM containing 10% FBS, 1x non-essential amino acids (NEAA) and antibiotics] containing doxycycline at a final concentration of 2 µg/mL, and the cells were cultured. The cells were washed with PBS 72 hours after the medium replacement, collected, and lysed in a 1×SDS sample buffer. Then, the sample was treated at 99°C for 10 minutes, and subjected to SDS-PAGE and Western blotting. For detection of Rep proteins, an anti-Rep mouse monoclonal 303.9 antibody or an anti-Rep mouse monoclonal 259.5 antibody (Progen) was used as the primary antibody. For detection of Cap proteins, an anti-Cap rabbit polyclonal antibody or an anti-Cap mouse monoclonal B1 antibody (Progen) was used as the primary antibody. For detection of beta-actin (β-actin) protein, an anti-β-actin rabbit polyclonal antibody (CST) was used as the primary antibody. As the secondary antibody, an anti-mouse IgG-HRP (Cell signaling technology) or an anti-rabbit IgG-HRP (Cell signaling technology) was used.

### 2-6. Measurement of Cap protein by immunoprecipitation

Anti-intact particle AAV Ab (ADK1a for AAV1 and AAV6; A20 for AAV2; ADK5a for AAV5; ADK8 for AAV7, AAV8 and AAVrh10; ADK8/9 for AAV9) and Protein A/B magnetic beads (Dynabeads) were reacted to prepare antibody-bead complexes. The antibody-bead complexes were washed using a magnetic stand, and resuspended in an IP buffer (cell lysis buffer). Then, the antibody-bead complexes were added to each of the virus solutions that were adjusted to have the same titer based on the qPCR data. After reaction at room temperature for 1 hour, AAV-antibody-bead complexes were collected using a magnetic stand, washed 3 to 4 times, and dissolved in a 1×SDS sample buffer. Then, the sample was treated at 99°C for 10 minutes and subjected to SDS-PAGE and Western blotting. For detection of Cap proteins, an anti-Cap rabbit polyclonal antibody or an anti-Cap mouse monoclonal B1 antibody (Progen) was used as the primary antibody, and an anti-rabbit IgG-HRP (Cell signaling technology) or a Clean-blot IP detection reagent (ThermoFisher) was used as the secondary antibody.

### 2-7. Measurement of Cap protein amount by ELISA

The amount of Cap proteins per 1 µL of each supernatant of the virus solutions obtained in 2-2 was measured using an AAV Titration ELISA Kit (Progen) according to the manufacturer's instructions.

### 2-8. Establishment of HEK293-Tet-Cap2 stable clone

HEK293 cells were transfected with pcDNA6-TR (Invitrogen) containing a Tet repressor. The transfected cells were selected with blasticidin to establish Tet repressor stable clones. Then, the Tet repressor stable clones were transfected with pcDNA4/TO-preCap2, and the transfected clones were selected with zeocin. Then, the responsiveness to doxycycline and the expression levels of Cap proteins determined by Western blotting were compared between the clones to establish HEK293-Tet-Cap2 stable clones.

### 2-9. Production of AAV vectors by Tet-Cap cell line system

To construct a cell line system for producing AAV vectors while adjusting the timing of Cap expression (Tet-Cap cell line system), the HEK293-Tet-Cap2 stable clones were transfected with pAAV-EGFP, pHelper, and pCMV-Rep2 in a ratio of pAAV-EGFP : pHelper : pCMV-Rep2 = 1 : 1 : 0.25, using HilyMax Transfection Reagent (DOJINDO LABORATORIES). Simultaneously with transfection, or 3, 6, 12 or 24 hours after transfection, a medium was replaced with a fresh medium [MEM containing 10% FBS, 1x non-essential amino acids (NEAA) and antibiotics] containing doxycycline at a final concentration of 2 µg/mL, and the cells were cultured for 72 hours after the medium replacement. The cells and a supernatant were collected together, and frozen at -80°C. The frozen cell/supernatant sample was thawed at 37°C, vortexed for 30 seconds, and then flash frozen in dry ice-ethanol. After such a freeze-thaw process was repeated a total of four times, the sample was centrifuged and a supernatant was collected. The supernatant was passed through a 0.45 µm filter to remove impurities, and the resulting solution was dispensed as a virus solution and stored.

### 2-10. Confirmation of AAV vector infectivity using mouse

In the Tet-Cap system (the method as described in 2-2), the conditions of "pAAV-EGFP-P2A-NanoLuciferase: pHelper: pTet-Cap9: pCMV-Rep2" were used to produce an AAV9 vector carrying a NanoLuciferase gene. In the conventional production system (control), an AAV9 vector carrying a NanoLuciferase gene was produced. The AAV9 vector (5×10¹⁰ vg) produced in each system was administered to C57BL/6 mice via their orbital floor vein. Four, six and eight weeks after administration, a Luciferase substrate solution prepared by dissolving 2.5 µL of NanoLuciferase substrate (Promega) in 100 µL of PBS was administered to each mouse. Thirty seconds to one minute after administration of the substrate solution, the amount of luciferase luminescence in each part of the mouse was measured using an in vivo luminescence/fluorescence imaging system (IVIS Spectrum CT; Perkin Elmer).

### (Results)

### 2-1. Effect of separate expression of Rep gene and Cap gene on AAV vector production

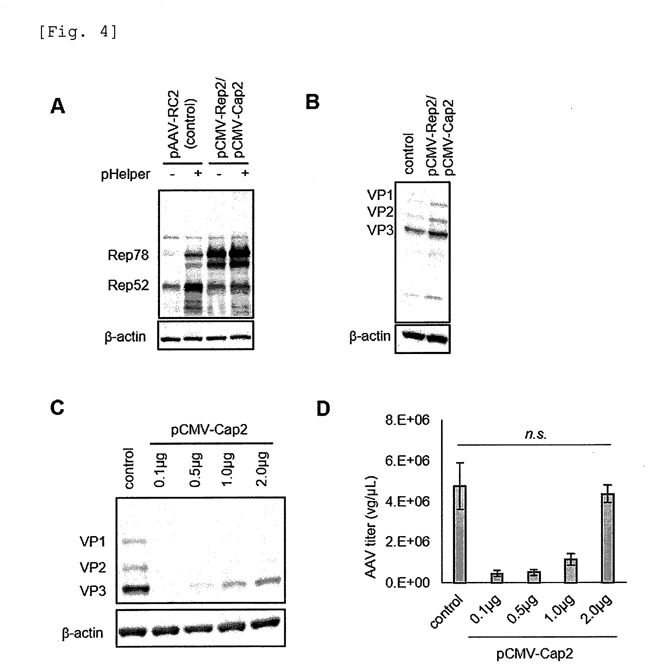

In order to adjust the Rep and Cap expression, the Rep gene and the Cap gene were separated, and plasmids containing each gene located so that the gene expression would be controlled by the CMV promoter were prepared (pCMV-Rep2 and pCMV-Cap2) . HEK293 cells were transfected with pAAV-EGFP, pHelper, pCMV-Cap2, and pCMV-Rep2 in a ratio of pAAV-EGFP : pHelper : pCMV-Cap2 : pCMV-Rep2 = 2 µg : 2ug : 2ug : 0.5pg, and compared with the control (pAAV-EGFP : pHelper : pAAV-RC2 : pcDNA3.1 = 2ug : 2ug : 2ug : 0.5µg). As a result, it was found that the cells transfected with pCMV-Rep2/pCMV-Cap2 had increased expression of Rep78 and decreased expression of Rep52 as compared with the control (FIG. 4A). On the other hand, the presence or absence of pHelper did not significantly change the expression patterns of Rep proteins in the cells transfected with pCMV-Rep2/pCMV-Cap2 (FIG. 4A). Furthermore, no significant change in the expression patterns of Cap proteins was observed between the cells transfected with pCMV-Rep2/pCMV-Cap2 and the control (FIG. 4B). To examine changes in the AAV vector production amount caused by adjustment of the expression level of Cap, the cells were transfected with different volumes of pCMV-Cap2. As a result, it was found that the expression of Cap proteins increased in a volume-dependent manner (FIG. 4C). When changes in the AAV vector production amount were examined under the same conditions, the production amount increased as the amount of pCMV-Cap2 increased (FIG. 4D). However, even when 2.0 µg of pCMV-Cap2 was used, the AAV vector production amount was almost the same as the AAV vector amount produced when the control was used. Furthermore, even when the cells were transfected with 2.0 µg or more of pCMV-Cap2, no improvement in the AAV vector production was observed (data not shown). These results suggest that it is difficult to increase the AAV vector production amount by simply separating the Rep gene and the Cap gene and adjusting the expression amount of Cap.

### 2-2. Improvement of AAV vector production by Tet-Cap system

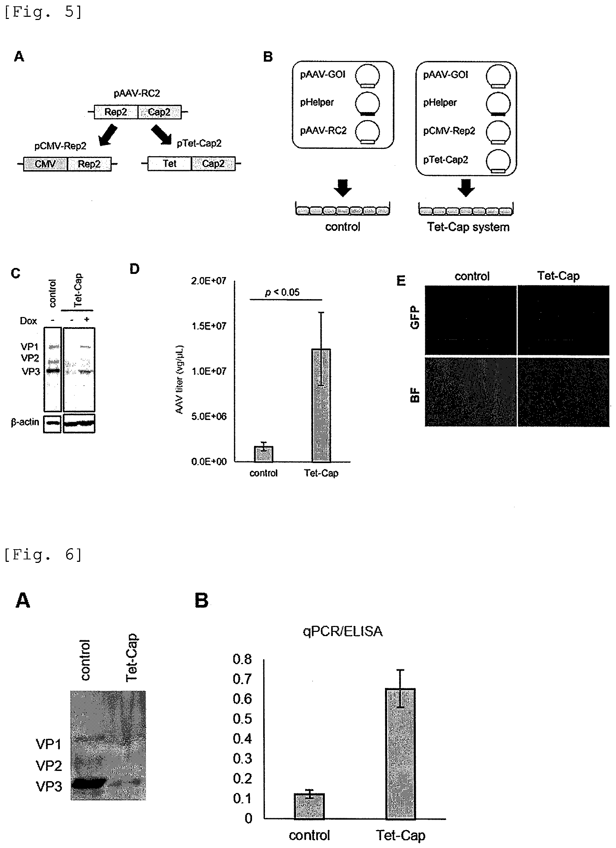

Various studies have reported that when Cap is expressed alone, the Cap proteins form VLPs in a variety of viruses. That is, in AAV, particle formation occurs immediately upon expression of Cap, and empty particles that do not contain the AAV genome are produced. Since Rep and Cap are expressed simultaneously in pAAV-RC, it is not possible to adjust the expression amounts and the expression timings of Cap and Rep. Thus, the Rep2 gene and the Cap2 gene of pAAV-RC2 were separated, and pCMV-Rep2 in which the Rep2 gene was introduced to the downstream of the CMV promoter and pTet-Cap2 in which expression of the Cap2 gene can be regulated in a doxycycline-dependent manner were prepared (Fig. 5A). The AAV vector production system using these plasmids (Tet-Cap system) was compared with the conventional production system (control) (Fig. 5B).

First, the Tet-Cap system was examined for the expression of Cap proteins. As a result, it was found that the expression of Cap proteins by the Tet-Cap system was regulated in a doxycycline-dependent manner, and the expression patterns of Cap proteins in the Tet-Cap system were not significantly different from those in the control (Fig. 5C). When the AAV vector amount produced by the Tet-Cap system was compared with that in the control, it was found that the Tet-Cap system increased the AAV vector production amount up to at least 7 times that of the control (Fig. 5D). Furthermore, for comparison of the infectivity of the AAV vectors produced by these systems, the AAV vector solutions obtained by these systems were adjusted to have the same titer based on the qPCR data. When 2V6.11 cells were infected with such AAV vector solutions, no significant difference in the infectivity was observed between the Tet-Cap system and the control (Fig. 5E). Thus, it was demonstrated that the Tet-Cap system enables efficient production of AAV vectors.

### 2-3. Increase in proportion of mature AAV by Tet-Cap system

The Tet-Cap system was shown to increase the AAV vector production amount. In order to show whether the Tet-Cap system increase a mature AAV proportion, the virus solutions adjusted to have the same titer based on the qPCR data were subjected to immunoprecipitation using an antibody specific for intact particles (including empty particles), and then to Western blotting to measure Cap proteins. As a result, the amounts of Cap proteins were decreased in the Tet-Cap system as compared with the control using pAAV-RC2 (Fig. 6A). Furthermore, the virus solution produced by each AAV vector production system was subjected to measurement of the Cap protein amount per unit amount by ELISA. To the Cap protein amount thus obtained, a proportion of the AAV vector amount calculated from qPCR data (qPCR/ELISA) was calculated to define a mature AAV proportion. As a result, it was found that the mature AAV proportion obtained by the Tet-Cap system was approximately 5 times higher than that obtained by the control (Fig. 6B). Thus, the Tet-Cap system significantly increases the mature AAV proportion.

### 2-4. Study of Tet-Cap system in other serotypes

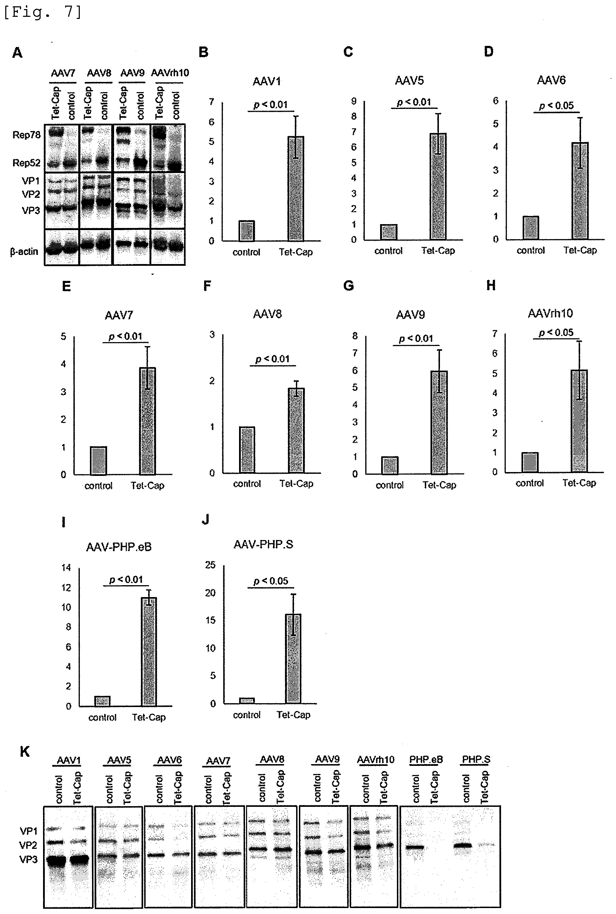

To confirm whether the improvement in the AAV vector production amount and the mature AAV proportion by the Tet-Cap system was observed similarly in other serotypes, pAAV-RC containing the Cap gene of each serotype and pTet-Cap containing the Cap gene of each serotype were prepared and used for experiments. After HEK293 cells were transfected with each of the prepared plasmids as well as other appropriate plasmids, the expression patterns of Rep proteins and Cap proteins were confirmed (Fig. 7A), and the production amounts of AAV vectors were measured (Fig. 7B-J). The Tet-Cap system produced a 2- to 5-fold increased amount of AAV vectors in all serotypes as compared with pAAV-RC (control) (Fig. 7B-J).

Furthermore, for comparison of the mature AAV proportions, the virus solutions adjusted to have the same titer were subjected to immunoprecipitation using an antibody specific for intact particles (including empty particles) . As a result, it was found that the amounts of Cap proteins were decreased in the virus group produced by the Tet-Cap system (Fig. 7K). Furthermore, when the infectivity of AAV vectors was compared, no significant difference was observed between pAAV-RC and pTet-Cap (data not shown). Thus, use of the Tet-Cap system for each serotype increased the AAV vector production amount and the mature AAV proportion without affecting the infectivity.

### 2-5. Effect of changed Rep expression pattern on AAV vector production

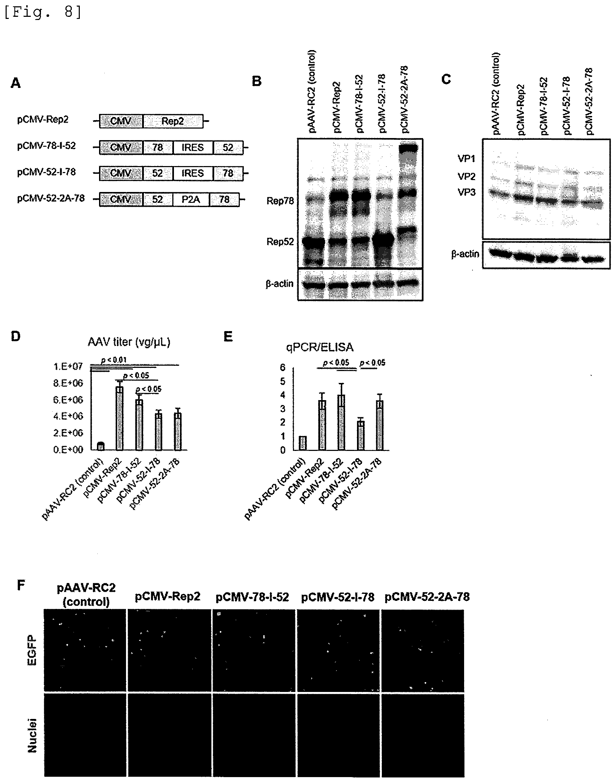

Next, how the Tet-Cap system improves the AAV vector production and the mature AAV proportion was analyzed. Various plasmids capable of expressing Rep78 and Rep52 were prepared and utilized in the Tet-Cap system (Fig. 8A). The expression patterns of Rep proteins were examined. As a result, when pAAV-RC2 (control) and pCMV-52-I-78 were used, the expression patterns of a high expression level of Rep52 and a low expression level of Rep78 were observed (Fig. 8B). On the other hand, when pCMV-Rep2 and pCMV-78-I-52 were used, the expression patterns of a high expression level of Rep78 and a low expression level of Rep52 were observed. When pCMV-52-2A-78 was used, Rep78 and Rep52 were produced in about equal amounts, while a fusion protein that did not undergo cleavage of the 2A peptide was also detected. No significant change in the Cap protein expression patterns was observed between the AAV vectors produced using each plasmid. Thus, it was found that the differences in the Rep protein expression patterns did not affect the Cap protein expression (Fig. 8C). The amounts of AAV vectors produced at this time were measured. As a result, it was found that pCMV-Rep2 and pCMV-78-I-52 increased the production amounts to a similar extent (Fig. 8D). On the other hand, pCMV-52-I-78 and pCMV-52-2A-78 produced less AAV vectors than pCMV-Rep2. The amounts of Cap proteins at this time were measured by ELISA, and the mature AAV proportions were calculated based on qPCR/ELISA. As a result, when pCMV-Rep2, pCMV-78-I-52 and pCMV-52-2A-78 were used, the mature AAV proportions were 3 to 5 times higher than that in pAAV-RC2 (control) (Fig. 8E). On the other hand, when pCMV-52-I-78 was used, the mature AAV proportion was relatively low, which was about twice that of the control. No difference in the infectivity was observed between the AAV vectors produced using each plasmid (Fig. 8F). These findings suggest that the change in the Rep expression patterns by the Tet-Cap system is one factor involved in increases of the AAV vector production amount and the mature AAV proportion.

### 2-6. Effect of adjustment of Cap expression timing on AAV vector production

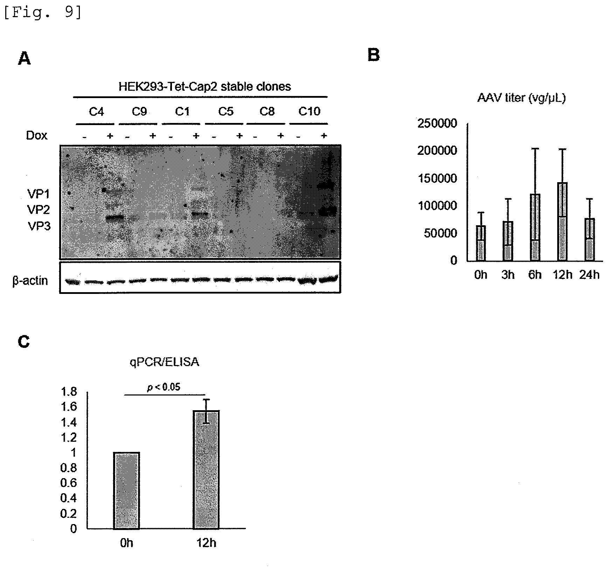

Next, in order to confirm the effect of adjusting the timing of Cap expression in the Tet-Cap system on increases of the AAV vector production and the mature AAV proportion, HEK293 cell lines stably retaining a Tet-Cap module that can induce the Cap expression at any timing by addition of doxycycline (HEK293-Tet-Cap2 stable clones) were established. The established cell lines were used to confirm the Cap protein expression control by doxycycline. As a result, it was found that the Cap protein expression was indeed induced by addition of doxycycline in some cell line clones (C4, C1, C10) (Fig. 9A). Next, these cell lines were transfected with pAAV-EGFP, pHelper and pCMV-Rep2, and then Cap proteins were induced by addition of doxycycline, thereby the production of AAV vectors was performed (Tet-Cap cell line system). No significant difference in the AAV vector production amount depending on different timings of doxycycline addition was observed (Fig. 9B, data of C4). On the other hand, when the Cap protein amounts were measured by ELISA and the mature AAV proportions were calculated based on qPCR/ELISA, the mature AAV proportion in the case of addition of doxycycline 12 hours after transfection was approximately 1.5-fold increased as compared with the case of addition of doxycycline simultaneously with transfection (0 h) (Fig. 9C). Thus, it was found that the mature AAV proportion is improved also by adjusting the timing of Cap expression.

### 2-7. Confirmation of AAV vector infectivity using mouse

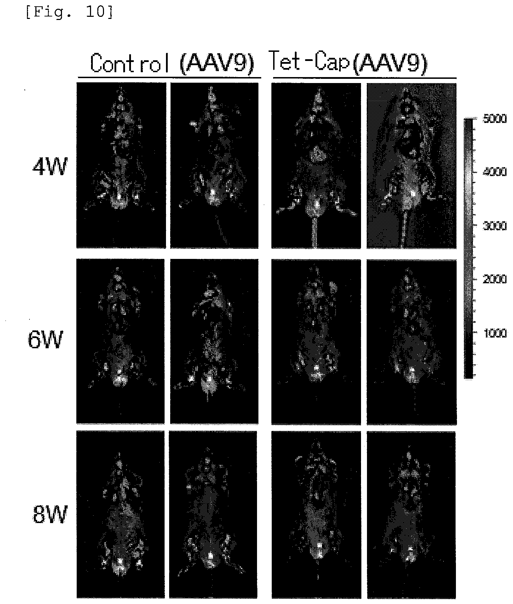

The infectivity of AAV vectors was examined by administering to mice the luciferase gene-carrying AAV9 vectors produced by using the Tet-Cap system or the conventional production system (control) (method 2-10). As a result, no significant changes in the infectivity and the organ tropism of the produced AAV vectors were observed between the conventional production system (control) and the Tet-Cap system (Fig. 10). Thus, it was demonstrated that the Tet-Cap system does not affect the infectivity and the tissue tropism of the produced AAV vectors.

### INDUSTRIAL APPLICABILITY

The methods of the present invention make it possible to obtain preparations containing AAV vectors with a high titer and a high proportion of mature AAV. The AAV vector prepared by the method of the present invention and a composition comprising the AAV vector as an active ingredient are extremely useful as a gene transfer method in the fields of basic research and clinical application of gene therapy.

### SEQUENCE LISTING FREE TEXT

SEQ ID NO: 1: YY1 binding sequence ACATNTT
SEQ ID NO: 2: YY1 binding sequence CCATNTT
SEQ ID NO: 3: 1xYY1 sequence
SEQ ID NO: 4: 2xYY1 sequence
SEQ ID NO: 5: 4xYY1 sequence
SEQ ID NO: 6: InsYY1+1 sense
SEQ ID NO: 7: InsYY1+1 Anti-sense
SEQ ID NO: 8: 2xYY1+1 sense
SEQ ID NO: 9: 2xYY1+1 Anti-sense
SEQ ID NO: 10: 4xYY1+1 sense
SEQ ID NO: 11: 4xYY1+1 Anti-sense
SEQ ID NO: 12: AAV2-ITR-qPCR-F
SEQ ID NO: 13: AAV2-ITR-qPCR-R
SEQ ID NO: 14: AAV2-preRep-5p
SEQ ID NO: 15: AAV2-preRep-3p
SEQ ID NO: 16: AAV2-preCap-5p
SEQ ID NO: 17: AAV2-preCap-3p
SEQ ID NO: 18: AAV2-Rep52-KZ-5p
SEQ ID NO: 19: AAV2-Rep52-Stop-3p
SEQ ID NO: 20: AAV2-Rep78-KZ-5p
SEQ ID NO: 21: AAV2-Rep78-Stop-3p
SEQ ID NO: 22: EcoRV-P2A-MfeI-EcoRI sense
SEQ ID NO: 23: EcoRV-P2A-MfeI-EcoRI Anti-sense
SEQ ID NO: 24: AAV2-Rep52-no-Stop-3p
SEQ ID NO: 25: AAV1,2,6,7,8,9,rh10-Cap-SwaI-5p
SEQ ID NO: 26: M13-For
SEQ ID NO: 27: AAV2,5,7,8,9,rh10 Cap-PmeI-3p
SEQ ID NO: 28: AAV1 Cap1-XbaI-XmaI-3p
SEQ ID NO: 29: AAV1 Cap1-EcoRV-3p-2
SEQ ID NO: 30: AAV7,8,9,rh10 preCap-EcoRV-3p
SEQ ID NO: 31: AAV9.47,PHP.eB,S Cap-SawI-5p
SEQ ID NO: 32: AAV9.47,PHP.eB,S Cap-XmaI-3p

## Claims

1. A nucleic acid construct comprising an inducible promoter and an adeno-associated virus (AAV) Cap protein-coding sequence operably linked to the promoter.

2. The nucleic acid construct according to claim 1, wherein the inducible promoter is a promoter to which a tetracycline response element sequence is operably linked.

3. A cell comprising the nucleic acid construct according to claim 1 or 2.

4. The cell according to claim 3, wherein the cell is derived from a human 293 cell.

5. A method for producing a recombinant AAV vector, the method comprising:
(a) culturing an adeno-associated virus (AAV) producer cell, wherein the AAV producer cell comprises a nucleic acid construct comprising an inducible promoter and an AAV Cap protein-coding sequence operably linked to the promoter;
(b) inducing the expression of the AAV Cap protein in the cell obtained by step (a); and
(c) culturing the cell obtained by step (b) to produce a recombinant AAV vector.

6. The method according to claim 5, wherein the inducible promoter is a promoter operably linked to a tetracycline response element sequence.

7. The method according to claim 5 or 6, wherein the step of inducing the expression of the AAV Cap protein is a step of contacting the cell with doxycycline or tetracycline.

8. The method according to claim 5, wherein step (b) is carried out 6 to 36 hours after the start of step (a).

9. The method according to claim 5, wherein step (b) is carried out 8 to 24 hours after the start of step (a).
